(19) Europäisches Patentamt
European Patent Office
Office européen des brevets

(11) EP 1 849 467 A1

(12) EUROPEAN PATENT APPLICATION
published in accordance with Art. 158(3) EPC

(43) Date of publication:
**31.10.2007 Bulletin 2007/44**

(21) Application number: **06705509.5**

(22) Date of filing: **20.01.2006**

(51) Int Cl.:
***A61K 31/352*** *(2006.01)* ***A61K 31/7048*** *(2006.01)*
***A61P 35/02*** *(2006.01)* ***A61P 35/04*** *(2006.01)*

(86) International application number:
**PCT/CN2006/000088**

(87) International publication number:
**WO 2006/081740 (10.08.2006 Gazette 2006/32)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.02.2005 CN 200510023724**
**29.04.2005 CN 200510025554**

(71) Applicant: **Shanghai Gloriayx Biopharmaceuticals Co., Ltd**
**Shanghai 201203 (CN)**

(72) Inventor: **WU Yixin**
**Shanghai 200051 (CN)**

(74) Representative: **Korga, Leokadia et al**
**Kancelaria Rzecznika Patentowego**
**ul. Bereniki 6/7**
**44-117 Gliwice (PL)**

# (54) THE SYNERGISTICALLY PHARMACEUTICAL COMPOSITION OF BAICALEIN AND BAICALIN FOR INHIBITING TUMOR

(57) Provided is a pharmaceutical composition with synergistic anti-tumor activities, comprising baicalein and a compound of formula I, **characterized in that** the molar ratio of baicalein and the compound of formula I is between 1:1 and 1:4,

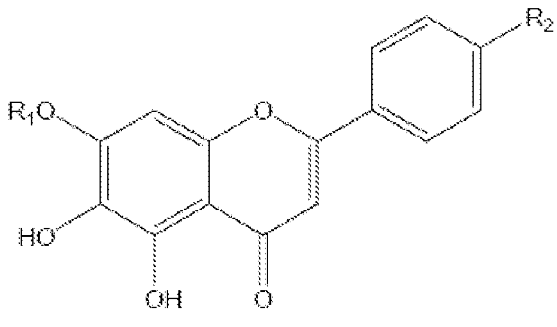

(I)

wherein R1 represents a glucuronic acid group, and R2 represents a hydrogen or hydroxyl group. Also disclosed is the use of the composition as well as a method of its preparation.

EP 1 849 467 A1

## Description

The invention relates to a pharmaceutical composition having synergistic anti-tumor effect, and more specifically to a pharmaceutical composition comprising flavonoids. The invention further relates to the preparation and pharmaceutical use of the composition.

Chemotherapy is a kind of treatment using specific chemical agents or drugs. Nowadays, the concept of chemotherapy is generally referred to tumor chemotherapy, namely, a method of treating tumor with the use of anti-tumor chemical agent and regiment. Currently, conventionally used chemotherapy agents, such as vincristine, cisplatin, aminopterin, cyclophosphamide, 5-fluorouracil (5-FU), etc., cause side effects like injection local pain, vein embolism, bone marrow depression, gastrointestinal tract reaction (GI tract discomfort), peripheral nerve disorder, etc.

In order to reduce side effects, increase therapeutic effect, lower tumor recurrence rate, and avoid the development of drug resistance, combination use of different chemotherapeutic agents becomes the main solution for tumor chemotherapy. For instance, the combination of cisplatin with 5-FU, bleomycin or epipodophyllotoxin has been used in clinical settings to treat esophageal carcinoma.

The term "synergistic effect" refers to such a situation in which the therapeutic effect of two combined medicaments is greater than the sum of their individual effects.

According to median-effect principle, (Joseph R. Bertino, Ting-Chao Chou, Chemotherapy: Synergism and Antagonism, Encyclopedia of Cancer, 1996, Academic Press, Inc.), the effect of two drugs used in combination can be determined by their "Combination Index" (CI) formula, as follows:

$$CI = \frac{D1}{(Dx)1} + \frac{D2}{(Dx)2} + \alpha \times \frac{D1 \times D2}{(Dx)1 \times (Dx)2}$$

where D1, D2 are the concentrations of drug 1 and 2 required to produce x% of the suppression ratio of cell proliferation alone, respectively; and (Dx)1, (Dx)2 are the concentrations in the mixture required to produce x% effect of suppression ratio of cell proliferation, respectively.

When the drugs are mutually exclusive (i.e. with similar modes of action) $\alpha$ = 0, or if they are mutually nonexclusive (i.e. with independent modes of action) $\alpha$ = 1.

A CI below 1 is an indication of synergy, while a CI equal to 1 represents addition, and a CI above 1 indicates antagonism.

According to the animal models for anti-tumor drug screening established by National Institute of Cancer Research (USA) in 1983, solid tumor cell such as murine melanoma cell line B16, mirune fibrosacroma cell line M5076, murine leukemia cell line L1210, etc can be used in vivo for mice transplantation study. Accordingly, skilled artisans will generally use a certain kind of tumor cell line to study the in vivo activities of an animal transplanted with the tumor cells. For instance, murine melanoma cell line B16 was used to set up murine solid tumor model and murine leukemia cell line L1210 was used to set up murine blood tumor model. The in vivo and in vitro experimental data obtain from these animal models can be used to determine if the substance to be tested has anti-tumor effect.

Scutellaria baicalensis Georgi, a traditional Chinese medicine, was first recorded in Shennong herbal materia medica. It is also named as Huangwen, Wuceng, etc. Scutellaria baicalensis Georgi is bitter in taste and cold in nature. It not only can discharge sthenic fire and remove damp heat, but also has the functions of bacteriostasis, heat abstraction, detoxication, sedation, decompression, benefit gallbladder, etc.

Baicalein and baicalin are the major flavonoid compounds contained in baikal skullcap root, both of which have the same flavone core structure of

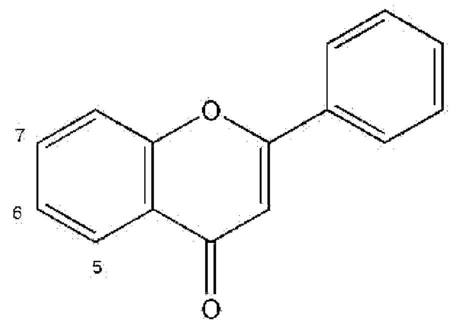

Baicalein is the major active component of Scutellaria baicalensis Georgi, with the molecular formula of C15H10O5, and molecular weight of 270.25. In positions 5, 6, and 7 of the flavone core structure, hydrogen (-H) is substituted by hydroxyl group (-OH).

The chemical structure of baicalin is as follows: In positions 5 and 6 of the flavone core structure, hydrogen (-H) is substituted by hydroxyl group (-OH), and in position 7, the hydroxyl group is condensed with glucuronic acid to form baicalein-7-O-glucuronic acid. The molecular formula of baicalin is C12H18O11, the molecular weight of which is 446.37.

Scutellarin also belongs to flavonoid compounds, which can be obtained from plants such as Portulaca. The molecular formula of scutellarin is C21 H19O12, with the molecular weight of 463 and the chemical structure of

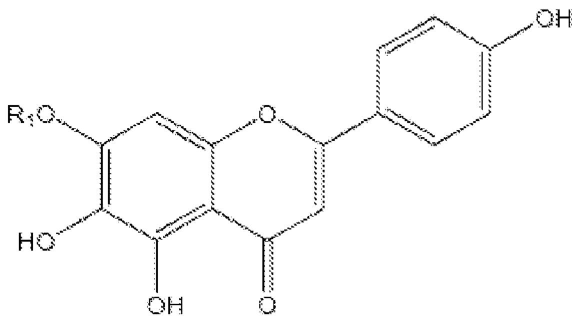

wherein R1 is a glucuronic acid group.

Hitherto, there is no report concerning the synergistic effect of baicalein and baicalin or scutellarin on anti-tumor treatment.

The invention is based on the finding that baicalein and baicalin or scutellarin exhibit synergistic effect, and the compositions of baicalein and baicalin or scutellarin have improved anti-tumor activity, which is higher than that of the compositionscontaining baicalein or baicalin/scutellarin alone.

In one aspect of the invention, there is provided a composition with synergistic anti-tumor activity. The composition comprises baicalein and a compound of formula I, characterized in that the molar ratio of baicalein and the compound of formula is between 1:1 and 1:4,

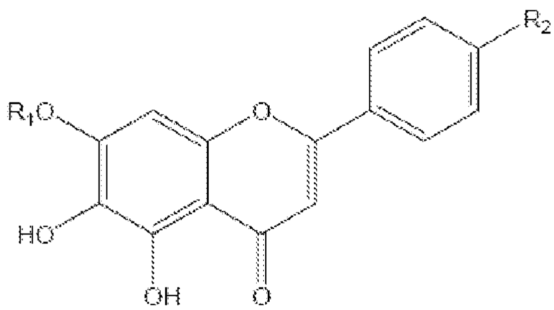

(I)

wherein $R_1$ is a glucuronic acid group; $R_2$ is a hydrogen or a hydroxyl group; and the tumor is a solid tumor or a blood tumor, and preferably, the tumor is melanoma, liver cancer, colon cancer, lung cancer, gastric cancer, esophageal cancer, breast cancer, prostate cancer or leukemia.

In another aspect of the invention, there is provided a method of preparing the composition, which comprises the step of mixing baicalein and at least one compound selected from formula I. In one embodiment, the method further includes the step of formulating the mixture into a suitable dosage form by adding pharmaceutically acceptable additives.

The invention further relates to the use of the composition in the preparation of a medicament for anti-tumor treatment, wherein the tumor is a solid tumor or blood tumor, and preferably, the tumor is melanoma, liver cancer, colon cancer, lung cancer, gastric cancer, esophageal cancer, breast cancer, prostate cancer or leukemia.

These and other objects of the invention, as well as many of the attendant advantages thereof, will become more readily apparent when reference is made to the following detailed description of the preferred embodiments.

It has been discovered that the combination use of baicalein and baicalin or baicalein and scutellarin showed synergistic effect, resulting in significantly improved anti-tumor activity. Accordingly, the dosages of baicalein and baicalin or scutellarin applied will be lowered, as will be the side effects. Therefore, the baicalein and baicalin or scutellarin of the invention can be used to prepare anti-tumor medicament having synergistic effect.

Baicalein, baicalin, and scutellarin of the invention exist in nature and can be extracted from plants such as Scutellaria baicalensis Georgi, Portulaca, etc. These compounds can also be prepared and purchased commercially, or produced from microorganism or chemical methods by a skilled technician. Chemical agents used to isolate or synthesize baicalein, baicalin, and scutellarin include solvents, reagents, catalysts, protective group reagents, and deprotecting reagents. Said isolation and synthesis can further include the step of adding or removing suitable protective groups to obtain desired compounds. The methods for chemical conversion and group protection (or deprotection) of baicalein, baicalin, and scutellarin of the invention are conventionally known in the art. See e.g., R. Larock, Comprehensive Organic Transformations, VCH Publishers (1989); T.W. Green and P.G.M. Wuts, Protective Groups in Organic Synthesis, 3rd Ed., John Wiley and Sons (1999), L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) as well as its later versions.

Baicalein and baicalin or scutellarin of the invention may be administrated simultaneously or separately, and the compositions of the invention containing baicalein and baicalin or scutellarin may be administrated parenterally or non-parenterally. For non-parental administration, the compositions may be in the forms of pills, granules, capsules, suspensions, or solutions. For parenteral administration, the compositions may be in the form of injectable suspensions, creams, ointments, patches, or sprays. The term "parenteral" as used herein, includes subcutaneous, intracutaneous, intravenous, intramuscular, intraarticular, intraarterial, intrasynovial, intrasternal, intrathecal, intralesional, and intracranial injection or infusion techniques. Other administration routes include topical, rectal, nasal, buccal, vaginal, sublingual, intradermal, mucosal, intratracheal, or intraurethral routes. The compositions of the invention may also be administered via inhalation spray or an implanted reservoir, or through an acupuncture point.

For parental administration, the dosage forms of the compositions include, but not limited to, capsules, tablets, emulsions and aqueous suspensions, dispersions, solutions, microcapsules, pills, lozenges, granules, and powders. For tablets, pharmaceutically acceptable carriers conventionally used include lactose and corn starch. Lubricating agents, such as magnesium stearate, are also typically included. For capsules, pharmaceutically acceptable carriers conventionally used include lactose and dried corn starch. When aqueous suspensions and/or emulsions are administered orally, the composition of the invention may be suspended or dissolved in an oily phase and combined with emulsifying and/or suspending agents. If desired, certain sweetening and/or flavoring and/or coloring agents may also be added.

The compositions of the invention may be in the form of a sterile injectable preparation, for example, a sterile injectable aqueous or oleaginous suspension. The suspension may be formulated according to techniques known in the art using suitable dispersing or wetting agents (such as Tween 80) and suspending agents. The sterile injectable preparation may also be a sterile injectable solution or suspension in a non-toxic parenterally acceptable diluent or solvent, for example, 1,3-butanediol. Among the acceptable vehicles and solvents that may be employed are mannitol, water, Ringer's solution, and isotonic sodium chloride solution, etc. In addition, sterilized fixed oils are conventionally employed as a solvent or suspending medium. For this purpose, any bland fixed oil may be employed, including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, or carboxymethyl cellulose or similar dispersing agents which are commonly used in the formulation of pharmaceutically acceptable dosage forms such as emulsions and or suspensions. Other commonly used surfactants such as Tweens or Spans and/or other similar emulsifying agents or bioavailability enhancers which are commonly used in the manufacture of pharmaceutically acceptable solid, liquid, or other dosage forms may also be used for the purposes of formulation.

The compositions of the invention may also be administered in the form of suppositories for rectal administration. For this purpose, the compositions were mixed with a suitable non-irritating excipient, which is solid at room temperature

but liquid at rectal temperature and therefore will melt in rectum to release active components. Such excipients include, but not limited to, cocoa butter, beeswax, and polyethylene. The topical formulation (e.g., ointment) containing the composition may be applied directly to suffered areas. Such topical formulation includes both active ingredients as well as a carrier, the latter of which includes, but not limited to, mineral oil, liquid petroleum, white petroleum, propylene glycol, polyoxyethylene or polyoxypropylene compound, emulsifying wax, and water. Alternatively, the composition can be formulated into lotion or cream, where the suitable carriers used include, but not limited to, mineral oil, sorbitan monostearate, polysorbate 60, cetyl esters wax, cetearyl alcohol, 2-octyldodecanol, benzyl alcohol, or water. The compositions may also be topically applied to the lower intestinal tract by rectal suppository formulation or in a suitable enema formulation. Topically transdermal patches are also included in this invention. The composition of the invention may also be administered by nasal aerosol or inhalation. Such compositions are prepared according to techniques well known in the art of pharmaceutical formulation and may be prepared as solutions in saline, employing benzyl alcohol or other suitable preservatives, absorption promoters, fluorocarbons, and/or other solubilizing or dispersing agents known in the art.

The compositions of the invention can be administered using an implantable device. Implantable devices and related technology are known in the art and are useful as delivery systems where a continuous or timed-release delivery of the compositions is desired. Additionally, the implantable device delivery system is useful for targeting specific points of delivery (e.g., localized sites and organs). See, e.g., Negrin et al., Biomaterials 22(6):563, 2001. Timed-release technology involving alternate delivery methods can also be used in this invention. For example, timed-release formulations based on polymer technologies, sustained-release techniques and encapsulation techniques (e.g., polymeric and liposomal) can also be used for delivery of the compositions of the invention.

Also within the scope of the invention is a patch used to deliver the compositions of the invention. The patch includes a material layer (e.g., polymeric, cloth, gauze, and bandage) and the compositions of the invention. One side of the material layer can has a protective layer adhered thereto to prevent the permeation of the active ingredients. The patch can additionally include an adhesive to hold the patch in place on a subject. The adhesive is a natural or synthesized composition, which will temporarily adhere to the skin of a subject upon contacting. The adhesive can be waterproof.

A "pharmaceutically acceptable carrier" will not destroy the pharmacological activities of compositions of the invention, and is non-toxic when administered in doses sufficient to deliver an effective amount of the extract or its ingredients. Pharmaceutically acceptable carriers that may be used include, but not limited to, ion exchangers, alumina, aluminum stearate, lecithin, self-emulsifying drug delivery systems (SEDDS) such as d-$\alpha$-tocopherol polyethyleneglycol 1000 succinate, surfactants used in pharmaceutical dosage forms such as Tweens or other similar polymeric delivery matrices, serum proteins such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, and magnesium trisilicate. polyvinyl pyrrolidone, cellulose-based substances, polyvinyl alcohol, sodium carboxymethylcellulose, polyacrylates, ethylene-polyoxypropylene-block polymers, and wool fat. Cyclodextrins such as $\alpha$-, $\beta$-, and $\gamma$-cyclodextrin, or chemically modified derivatives such as hydroxyalkylcyclodextrins, including 2- and 3-hydroxypropyl-$\beta$-cyclodextrins, or other solubilized derivatives may also be advantageously used to enhance the delivery of the pharmaceutical composition of the invention.

The effective molar ratio of baicalein and baicalin/scutellarin used in the composition of the invention so as to obtain synergistic effect has been determined and tested through appropriate in vitro assays. The dosage of baicalein and baicalin used as apoptosis inducer was disclosed in WO93/23033, which may be adjusted according to administration route and the age and conditions of the subject to be treated. Generally, for oral administration, the dosage of baicalein or baicalin is 100-6000 mg/day/person, 1-3 times a day. For non-oral administration, the dosage of baicalein and baicalin can be 1-100 mg/day/person. Until now, there have been no reports concerning the dosage and the administration route of scutellarin in anti-tumor treatment.

With conventional techniques in the art, a skilled artisan would understand how to combine baicalein and baicalin/scutellarin according to the molar ratio disclosed herein to prepare the synergistic anti-tumor pharmaceutical composition of the invention.

The following examples are included for illustrative purposes only and are not intended to limit the scope of the invention.

EXAMPLES

Example 1

Synergistic growth inhibition effect in various cultured human cancer cell lines by a combination of baicalein and baicalin.

Cells were seeded onto a 96-well plate at $5\times10^3$ cells/well, and grown in culture medium containing 10% fetal bovine serum (FBS). After 24-h incubation, the cells were treated with single or combination dosing of baicalein (Kunming Tongchi Pharmaceutical Co., Ltd.) and baicalin (Sichuan Chengdu Superman Plant Chemical Development Co., Ltd) at various concentrations. The viable cell growth was determined by MTT assay after 72-h continuous exposure. The

percentage of growth inhibition in single or combination dosing of baicalein and baicalin were determined. Combination indices (CI) were calculated by CalcuSyn software for each combination.

A CI below 1 is an indication of synergy, while a CI equal to 1 represents addition, and a CI above 1 indicates antagonism.

Growth inhibition rate in single and combination dosing of baicalein and baicalin, and combination indices(CI), see table1-8.

Various human cancer cell lines were used and the following were the results;

The results of experiments with human liver cancer cell line HepG2 are illustrated in Table 1;

Human colon cancer cell line HCT116 - Table 2;

Human hung cancer cell line A549 - Table 3;

Human gastric cancer cell line MKN28 - Table 4;

Human esophagus cancer cell line TE2 - Table 5;

Human breast cancer cell line MCF-7 - Table 6;

Human prostate cancer cell line PC3 - Table 7; and

Human leukemia cell line HL60 - Table 8.

Table 1

| Single dose (μm) | Combination dose(μm) | Inhibition of cell growth (%) | Combination Index(CI) |
|---|---|---|---|
| B 2.5 | | 7.4±2 | |
| B 5 | | 34.93±3 | |
| B 10 | | 50.2±3 | |
| B 20 | | 58.5±2.5 | |
| B 40 | | 89.18±2 | |
| BG 2.5 | | 16.63±2 | |
| BG 5 | | 16.8±2 | |
| BG 10 | | 34.93±3.8 | |
| BG 20 | | 50±2.9 | |
| BG 40 | | 86.69±3 | |
| 1:1 | B 5+BG 5 | 45±5 | 0.95 |
| | B 10+BG 10 | 70±3 | 0.88 |
| | B 20+BG 20 | 97.5±3 | 0.228 |
| 1:2 | B 2.5+BG 5 | 9.64±1 | 3.26 |
| | B 5+BG 10 | 66.1±3 | 0.7143 |
| | B 10+BG 20 | 80.9±2 | 0.7961 |
| | B 20+BG 40 | 92.3±2 | 0.7299 |
| 1:4 | B 2.5+BG 10 | 38.43±3 | 1.379 |
| | B 5+BG 20 | 83.2±2 | 0.5542 |
| | B 10+BG 40 | 91.7±2.1 | 0.5965 |

Table 2

| Single dose (μm) | Combination dose (μm) | Inhibition of cell growth (%) | Combination Index (CI) |
|---|---|---|---|
| B 2.5 | | 5.8±2 | |
| B 5 | | 20.5±3 | |
| B10 | | 29.2±3 | |

(continued)

| Single dose (μm) | Combination dose (μm) | Inhibition of cell growth (%) | Combination Index (CI) |
|---|---|---|---|
| B 20 | | 41 ±2.7 | |
| B 40 | | 89.15±3 | |
| BG 2.5 | | 17±2 | |
| BG 5 | | 20±3.2 | |
| BG 10 | | 27±3 | |
| BG 20 | | 33.75±3 | |
| BG 40 | | 90±2 | |
| 1:1 | B 5+BG 5 | 32.25±3 | 1.15 |
| | B 10+BG 10 | 63.45±3.2 | 0.88 |
| | B 20+BG 20 | 95±1 | 0.31 |
| 1:2 | B 2.5+BG 5 | 12.8±3.7 | 2.22 |
| | B 5+BG 10 | 35.9±3 | 1.56 |
| | B 10+BG 20 | 72.5±3 | 0.94 |
| | B 20+BG 40 | 97±1 | 0.285 |
| 1:4 | B 2.5+BG 10 | 29.2±2.9 | 1.68 |
| | B 5+BG 20 | 60.62±3 | 1.17 |
| | B 10+BG 40 | 96.71 ±2.1 | 0.2322 |

Table 3

| Singl e dose (μm) | Combination dose (μm) | Inhibition of cell growth (%) | Combination Index (CI) |
|---|---|---|---|
| B 2.5 | | 13.5±4 | |
| B 5 | | 17.18±3 | |
| B 10 | | 39.2±2 | |
| B 20 | | 63.85±3.2 | |
| B 40 | | 96.41±2 | |
| BG 2.5 | | 9.8±2 | |
| BG 5 | | 17±3 | |
| BG 10 | | 30.58±3.2 | |
| BG 20 | | 53.58±4 | |
| BG 40 | | 92±3 | |
| 1:1 | B 5+BG 5 | 58±2 | 0.734 |
| | B 10+BG 10 | 85.5±2 | 0.626 |
| | B 20+BG 20 | 98±3 | 0.364 |
| 1:2 | B 2.5+BG 5 | 15.8±3 | 1.742 |
| | B 5+BG 10 | 78.3±2.2 | 0.54 |
| | B 10+BG 20 | 86.03±1.95 | 0.8588 |
| | B 20+BG 40 | 96.5±1 | 0.702 |
| 1:4 | B 2.5+BG 10 | 49.55±2 | 0.9 |

(continued)

| Singl e dose (μm) | Combination dose (μm) | Inhibition of cell growth (%) | Combination Index (CI) |
|---|---|---|---|
| | B 5+BG 20 | 96.03±1 | 0.295 |
| | B 10+BG 40 | 97.2±1 | 0.457 |

Table4

| Single dose (μm) | Combination dose (μm) | Inhibition of cell growth (%) | Combination Index (CI) |
|---|---|---|---|
| B 2.5 | | 7.7±3 | |
| B 5 | | 37.11±2.9 | |
| B 10 | | 53±3.2 | |
| B 20 | | 58±2 | |
| B 40 | | 77±3 | |
| BG 2.5 | | 5.2±2 | |
| BG 5 | | 12±3 | |
| BG 10 | | 30.2±2.9 | |
| BG 20 | | 62±3.2 | |
| BG 40 | | 85.22±2 | |
| 1:1 | B 5+BG 5 | 41±3 | 0.95 |
| | B 10+BG 10 | 67.8±2.2 | 0.853 |
| | B 20+BG 20 | 85.7±2.3 | 0.815 |
| 1:2 | B 2.5+BG 5 | 12±3 | 2.13 |
| | B 5+BG 10 | 35.2±5 | 1.61 |
| | B 10+BG 20 | 76.5±2.5 | 0.95 |
| | B 20+BG 40 | 93±2 | 0.76 |
| 1:4 | B 2.5+BG 10 | 25±3 | 1.76 |
| | B 5+BG 20 | 70.7±3 | 0.98 |
| | B 10+BG 40 | 95±2 | 0.536 |

Table5

| Single dose (μm) | Combination dose (μm) | Inhibition of cell growth (%) | Combination Index (CI) |
|---|---|---|---|
| B 2.5 | | 5.5±1 | |
| B 5 | | 17.2±2.8 | |
| B 10 | | 28±2 | |
| B 20 | | 57.9±2.1 | |
| B 40 | | 85.2±2.3 | |
| BG 2.5 | | 6.2±1.8 | |
| BG 5 | | 12±3 | |
| BG 10 | | 28.2±2.5 | |
| BG 20 | | 49.8±1.78 | |

(continued)

| Single dose (μm) | Combination dose (μm) | Inhibition of cell growth (%) | Combination Index (CI) |
|---|---|---|---|
| BG 40 | | 72.7±2.1 | |
| 1:1 | B 5+BG 5 | 18.7±2.3 | 1.57 |
| | B 10+BG 10 | 47.1±2.9 | 1.259 |
| | B 20+BG 20 | 81.2±2 | 0.87 |
| 1:2 | B 2.5+BG 5 | 10.1±2 | 1.91 |
| | B 5+BG 10 | 29.12±2.1 | 1.545 |
| | B 10+BG 20 | 65.31±3 | 0.85 |
| | B 20+BG 40 | 92±3 | 0.617 |
| 1:4 | B 2.5+BG 10 | 7.8±1 | 3.89 |
| | B 5+BG 20 | 59±3 | 0.99 |
| | B 10+BG 40 | 93±2 | 0.428 |

Table 6

| Singl e dose (μm) | Combination dose (μm) | Inhibition of cell growth (%) | Combination Index (CI) |
|---|---|---|---|
| B 2.5 | | 7.2±2 | |
| B 5 | | 27.55±2 | |
| B 10 | | 51±3 | |
| B 20 | | 71.77±2.5 | |
| B 40 | | 90.5±2.5 | |
| BG 2.5 | | 0.5±2.7 | |
| BG 5 | | 10.2±1 | |
| BG 10 | | 48.02±2 | |
| BG 20 | | 60.35±3 | |
| BG 40 | | 90±2 | |
| 1:1 | B 5+BG 5 | 56.04±3 | 0.725 |
| | B 10+BG 10 | 88.95±2 | 0.573 |
| | B 20+BG 20 | 95.53±1 | 0.711 |
| 1:2 | B 2.5+BG 5 | 49.43±2 | 0.587 |
| | B 5+BG 10 | 80±2 | 0.6 |
| | B 10+BG 20 | 91.5±1.5 | 0.762 |
| | B 20+BG 40 | 97.7±1 | 0.82 |
| 1:4 | B 2.5+BG 10 | 68.28±2.22 | 0.6529 |
| | B 5+BG 20 | 89.4±1 | 0.78 |
| | B 10+BG 40 | 97±1 | 0.8 |

Table 7

| Singl e dose (μm) | Combination dose (μm) | Inhibition of cell growth (%) | Combination Index (CI) |
|---|---|---|---|
| B 2.5 | | 7.2±5 | |
| B 5 | | 22.8±6 | |
| B 10 | | 50.9±5 | |
| B 20 | | 69.9±5 | |
| B 40 | | 92.9±7 | |
| BG 2.5 | | 1±3 | |
| BG 5 | | 17.9±5 | |
| BG 10 | | 47±8 | |
| BG 20 | | 62±7 | |
| BG 40 | | 88±7 | |
| 1:1 | B 5+BG 5 | 47.9±5 | 0.87 |
| | B 10+BG 10 | 77±3 | 0.89 |
| | B 20+BG 20 | 92±5 | 0.95 |
| 1:2 | B 2.5+BG 5 | 45±3 | 0.66 |
| | B 5+BG 10 | 72.8±5 | 0.73 |
| | B 10+BG 20 | 87±3 | 0.92 |
| | B 20+BG 40 | 95±5 | 1.1 |
| 1:4 | B 2.5+BG 10 | 55.8±6 | 0.85 |
| | B 5+BG 20 | 77.9±5 | 1 |
| | B 10+BG 40 | 97±5 | 0.72 |

Table 8

| Single dose (μm) | Combination dose (μm) | Inhibition of cell growth (%) | Combination Index (CI) |
|---|---|---|---|
| B 2.5 | | 6.5±2 | |
| B 5 | | 21.8±3 | |
| B 10 | | 41.32±2.7 | |
| B 20 | | 67.6±3.2 | |
| B 40 | | 86.77±3.3 | |
| BG 2.5 | | 5±2 | |
| BG 5 | | 8.6±3 | |
| BG 10 | | 21±4 | |
| BG 20 | | 45.1±2.9 | |
| BG 40 | | 82.7±2.3 | |
| 1:1 | B 5+BG 5 | 43.13±3.23 | 0.79 |
| | B 10+BG 10 | 76.2±3 | 0.645 |
| | B 20+BG 20 | 95±2 | 0.426 |
| 1:2 | B 2.5+BG 5 | 10±2.5 | 1.81 |
| | B 5+BG 10 | 76.5±3 | 0.4445 |

(continued)

| Single dose (μm) | Combination dose (μm) | Inhibition of cell growth (%) | Combination Index (Cl) |
|---|---|---|---|
| | B 10+BG 20 | 85.2±2 | 0.62 |
| | B 20+BG 40 | 95±2 | 0.59 |
| 1:4 | B 2.5+BG 10 | 40.05±3 | 0.929 |
| | B 5+BG 20 | 78±2 | 0.66 |
| | B 10+BG 40 | 95±3 | 0.465 |
| B=baicalein; BG=baicalin | | | |

Example 2

Synergistic anti-tumor effects in a combination of baicalein and baicalin on solid tumor transplant animal models.

C57BL/6 female mice (weight approximately 20 g) were used in this study. Murine melanoma cell line B16 cells, $2\times10^6$tumor cells were implanted s.c. into the armpit of each mice after two passenges in vitro cultured. On the next day after implantation, mice were randomly grouped at 10 per group and received 0.1 ml/10g weight of the drugs. Anti-tumor effect studies were evaluated using a combination dosing of baicalein (Kunming Tongchi Pharmaceutical Co., Ltd.) and baicalin (Sichuan Chengdu Superman Plant Chemical Development Co., Ltd). The number of mice of each group was 10. Mice were weighed before and post the treatment. Animals were killed on day 15, and the tumors were removed and weighted. The tumor growth inhibition was determined as; inhibition rate=(1-tumor weight of treatment group/ tumor weight of control group) ×100%, tumor growth inhibition rate was calculated, the data were analyzed by the t-test. The results is shown in Table 9

Table 9

| Group | Dose mg/kg | Route and Schedule | Weight Change % | Final Tumor WT.(g) Mean±SD, | Tumor Growth Inhibition % | Number of mice Surviving Through 15days/total number |
|---|---|---|---|---|---|---|
| Control | - | | <0 | 2.18±0.15 | | 10/10 |
| B | 30 | i.p.; day1-10 | <5 | 1.72±0.09 | 21.1 | 10/10 |
| BG | 25 | i.p.; day1-10 | <5 | 1.81±0.1 | 16.97 | 10/10 |
| BG | 50 | i.p.; day1-10 | <5 | 1.77±0.117 | 18.8 | 10/10 |
| BG | 100 | i.p.; day1-10 | <5 | 1.51±0.11 | 30.7 | 10/10 |
| BG | 200 | i.p.; day1-10 | <5 | 1.28±0.07 | 41.28 | 10/10 |
| B+ | 30+ | i.p.; day1-10 | <5 | 1.65±0.09 | 24.3* # | 10/10 |
| BG | 25 | i.p.; day1-10 | | | | |
| B+ | 30+ | i.p.; day1-10 | <5 | 1.58±0.09 | 27.5** # | 10/10 |
| BG | 50 | i.p.; day1-10 | | | | |
| B+ | 30+ | i.p.; day1-10 | <5 | 1.29±0.1 1 | 40.8** # | 10/10 |
| BG | 100 | i.p.; day1-10 | | | | |
| B+ | 30+ | i.p.; day1-10 | <5 | 1.11±0.11 | 49** # | 10/10 |
| BG | 200 | i.p.; day1-10 | | | | |
| 5-FU | 10 | s.c.; day1-10 | <5 | 1.35±0.1 | 38 | 10/10 |

B=baicalein, BG=baicalin
*P<0.1 comparison between the baicalein-treated and a combination-treated group,
** P<0.01 comparison between the baicalein-treated and a combination-treated group.
# P<0.01 comparison between the baicalin-treated and a combination-treated group.

The study showed that significantly enhancement of anti-tumor activity by using baicalein and baicalin combination. Treatment group of a combination of baicalein and baicalin at various doses, such as baicalein+baicalin, 30+50 mg/kg; baicalein+baicalin, 30+100 mg/kg; baicalein+baicalin, 30+200 mg/kg demonstrated a significant statistical difference from experiments when ether baicalein or baicalin was used alone. In addition, the group of baicalein+baicalin

at a dose of 30+25mg/kg, also showed some enhancement of anti-tumor activity, but not clearly better than the other three dose groups. Animal weight and weight change indicated there was no enhancement of toxicity in each combination dosing group.

The ratio of baicalein to baicalin effect between 1:0.5 and 1:4 (molar ratio) started to show the synergistic anti-tumor effects, and reached a plateau between 1:1 and 1:4. The animal model data were consistent with those of in vitro studies. Therefore, a combination baicalein and baicalin has synergistic anti-tumor effects on solid tumors.

Example 3

Synergistic anti-tumor effects of baicalein and baicalin combination on transplant blood tumor modles.

L1210 cells on the growth phase were injected at $1 \times 10^5$ cells via I.P. into BDF1 female mice (weight approximately 20 g). The next day of implantation, mice were grouped, and received an injection of 0.1 ml/10g weight of the drug. Anti-tumor effects were evaluated using single or combination dosing of baicalein (Kunming Tongchi Pharmaceutical Co., Ltd.) and baicalin (Sichuan Chengdu Superman Plant Chemical Development Co., Ltd). The number of mice of each group was 10. Mice were weighed before and after the treatment. Survival time of each mouse was recorded. The results of changes in increasing of life span of mice were used in assessment of response. The increase of life span was determined as; increase of life span rate=(median survival time of treated animals/ median survival time of control animals-1)$\times$100%, the data were analyzed by t-test. The results is shown in Table 10

Table 10

| Group | Dose mg/kg | Route and Schedule | ILS(%) | Number of mice Surviving through 15days/total number |
|---|---|---|---|---|
| Control | - | | | 10/10 |
| B | 30 | i.p.; day1-10 | 31.6 | 10/10 |
| BG | 25 | i.p.; day1-10 | 17.3 | 10/10 |
| BG | 50 | i.p.; day1-10 | 27.55 | 10/10 |
| BG | 100 | i.p.; day1-10 | 38.27 | 10/10 |
| BG | 200 | i.p.; day1-10 | 50 | 10/10 |
| B+ | 30+ | i.p.; day1-10 | 37.2* # | 10/10 |
| BG | 25 | i.p.; day1-10 | | |
| B+ | 30+ | i.p.; day1-10 | 47.9**# | 10/10 |
| BG | 50 | i.p.; day1-10 | | |
| B+ | 30+ | i.p.; day1-10 | 55.1**# | 10/10 |
| BG | 100 | i.p.; day1-10 | | |
| B+ | 30+ | i.p.; day1-10 | 67** # | 10/10 |
| BG | 200 | i.p.; day1-10 | | |
| 5-FU | 10 | s.c.; day1-10 | 79 | 10/10 |

B=baicalein, BG=baicalin
ILS=increase of life span
*P<0.1 comparison between the baicalein-treated and a combination-treated group,
** P<0.01 comparison between the baicalein-treated and a combination-treated group.
# P<0.01 comparison between the baicalin-treated and a combination-treated group.

The study showed that significantly enhancement of anti-tumor activity by using baicalein and baicalin combination. Treatment group of a combination of baicalein and baicalin at various doses, such as baicalein+baicalin, 30+50 mg/kg; baicalein+baicalin, 30+100 mg/kg; baicalein+baicalin, 30+200 mg/kg demonstrated a significant statistical difference when compared to using ether baicalein or baicalin alone. In addition, the group of baicalein+baicalin at a dose of 30+25mg/kg, also showed some enhancement of anti-tumor activity, but not clearly better than other three dose groups. Date of animal weight, and weight change of indicated there was no enhancement of toxicity in each combination dosing group.

The ratio of baicalein to baicalin effect between 1:0.5 and 1:4 (molar ratio) started to show the synergistic anti-tumor effects, and reach the plateau between 1:1 and 1:4. The animal model data were consistent with those of in vitro studies. Therefore, a combination baicalein and baicalin has synergistic anti-tumor effects on blood tumor.

Example 4

Synergistic growth inhibition effect in various cultured human cancer cell lines by a combination of baicalein and scutellarin

Cells were seeded onto 96-well plate at $5 \times 10^3$ cells/well, and grown in culture medium containing 10% fetal

bovine serum (FBS). After 24-h incubation, the cells were treated with single or combination dosing of baicalein (Kunming Tongchi Pharmaceutical Co., Ltd.) and scutellarin (Kunming Longjin Pharmaceutical Co., Ltd) at various concentrations. The viable cell growth was determined by MTT assay after 72-h continuous exposure. The percentage of growth inhibition in single or combination dosing of baicalein and scutellarin were determined. Combination indices (Cl) were calculated by CalcuSyn software for each combination.

A Cl below 1 is an indication of synergy, while a Cl equal to 1 represents addition, and a Cl above 1 indicates antagonism.

Growth inhibition rate in single and combination dosing of baicalein and scutellarin, and combination indices (Cl), see table11-18.

Various human cancer cell lines were used and the following were the results.

The results of experiments with human liver cancer cell line HepG2 are illustrated in Table 11;

Human colon cancer cell line HCT116 - Table 12;

Human hung cancer cell line A549 - Table 13;

Human gastric cancer cell line MKN28 - Table 14;

Human esophagus cancer cell line TE2 - Table 15;

Human breast cancer cell line MCF-7 - Table 16;

Human prostate cancer cell line PC3 - Table 17; and

Human leukemia cell line HL60 - Table 18.

Table 11

| Single dose (μm) | Combination dose (μm) | Inhibition of cell growth (%) | Combination Index (Cl) |
|---|---|---|---|
| B 2.5 | | 7.35±2.2 | |
| B 5 | | 32.7±2.3 | |
| B 10 | | 49.8±2.1 | |
| B 20 | | 58.5±1.2 | |
| B 40 | | 90.18±2.5 | |
| Sc 2.5 | | 5.2±2.1 | |
| Sc 5 | | 7.7±2.3 | |
| Sc 10 | | 43.35±2.7 | |
| Sc 20 | | 86.1±2.9 | |
| Sc 40 | | 88.45±2.5 | |
| | B 5+Sc 5 | 70.1±2.2 | 0.537 |
| | B 10+Sc 10 | 85.2±2.5 | 0.647 |
| | B 20+Sc 20 | 92.77±3 | 0.82 |
| | B 40+Sc 40 | 97±3 | 0.987 |
| | B 2.5+Sc 5 | 52.9±2 | 0.6 |
| | B 5+Sc 10 | 88.12±2.1 | 0.438 |
| | B 10+Sc20 | 96.32±3.5 | 0.448 |
| | B 20+Sc 40 | 98±3 | 0.64 |
| | B 2.5+Sc 10 | 70.1±3 | 0.68 |
| | B 5+Sc 20 | 87.7±2.2 | 0.768 |
| | B 10+Sc 40 | 97±2.1 | 0.7 |
| | B 20+Sc 80 | 99±2 | 0.8 |

Table 12

| Single dose (μm) | Combination dose (μm) | Inhibition of cell growth (%) | Combination Index (CI) |
|---|---|---|---|
| B 2.5 | | 5.8±2 | |
| B 5 | | 10.5±2.3 | |
| B 10 | | 29.2±2.7 | |
| B 20 | | 47±3.1 | |
| B 40 | | 91.15±2.5 | |
| Sa 2.5 | | 10.05±2.5 | |
| Sc 5 | | 15.1±3 | |
| Sc 10 | | 49±2.2 | |
| Sc 20 | | 83.7±2.2 | |
| Sc 40 | | 95±2.8 | |
| | B 5+Sc 5 | 69.5±1.9 | 0.559 |
| | B 10+Sc 10 | 87.2±2.3 | 0.627 |
| | B 20+Sc 20 | 96±2.2 | 0.646 |
| | B 40+Sc 40 | 98.7±2.1 | 0.88 |
| | B 2.5+Sc 5 | 55±3 | 0.63 |
| | B 5+Sc 10 | 85.7±3.2 | 0.55 |
| | B 10+Sc 20 | 95±3.1 | 0.6 |
| | B 20+Sc 40 | 98.2±2.7 | 0.7 |
| | B 2.5+Sc 10 | 75.78±3.2 | 0.68 |
| | B 5+Sc 20 | 91.2±2.3 | 0.738 |
| | B 10+Sc 40 | 97±2.1 | 0.822 |
| | B 20+Sc 80 | 99±2 | 0.92 |

Table 13

| Single dose (μm) | Combination dose (μm) | Inhibition of cell growth (%) | Combination Index (CI) |
|---|---|---|---|
| B 2.5 | | 10.55±1.2 | |
| B 5 | | 17.19±2.2 | |
| B 10 | | 40.8±3.2 | |
| B 20 | | 62.5±2 | |
| B 40 | | 90.18±2.1 | |
| Sc 2.5 | | 1±2.1 | |
| Sc 5 | | 7.7±3.3 | |
| Sc 10 | | 32.33±2.7 | |
| Sc 20 | | 62.65±2.5 | |
| Sc 40 | | 91±2.7 | |
| | B 5+Sc 5 | 67.15±2.1 | 0.512 |
| | B 10+Sc 10 | 83.2±2.2 | 0.64 |
| | B 20+Sc 20 | 95±3.5 | 0.65 |

(continued)

| Single dose (μm) | Combination dose (μm) | Inhibition of cell growth (%) | Combination Index (CI) |
|---|---|---|---|
| | B 40+Sc 40 | 98±1.2 | 0.8 |
| | B 2.5+Sc 5 | 54.5±2 | 0.49 |
| | B 5+Sc 10 | 86.2±2.2 | 0.44 |
| | B 10+Sc 20 | 95.1±2.5 | 0.516 |
| | B 20+Sc 40 | 98.5±2 | 0.587 |
| | B 2.5+Sc 10 | 77±3 | 0.5 |
| | B 5+Sc 20 | 90.2±1.5 | 0.632 |
| | B 10+Sc 40 | 97±2 | 0.723 |
| | B 20+Sc 80 | 99±2 | 0.88 |

Table 14

| Single dose (μm) | Combination dose (μm) | Inhibition of cell growth (%) | Combination Index (CI) |
|---|---|---|---|
| B 2.5 | | 16.5±1.1 | |
| B 5 | | 27.7±2.1 | |
| B 10 | | 42.8±2.3 | |
| B 20 | | 65.6±3 | |
| B 40 | | 80.21±2.5 | |
| Sc 2.5 | | 18.83±2.2 | |
| Sc 5 | | 21.23±3.5 | |
| Sc 10 | | 31.65±2.8 | |
| Sc 20 | | 35.34±2.8 | |
| Sc 40 | | 75.34±2.7 | |
| | B 5+Sc 5 | 55±1.7 | 0.55 |
| | B 10+Sc 10 | 69±2.5 | 0.607 |
| | B 20+Sc 20 | 79±3.1 | 0.722 |
| | B 40+Sc 40 | 90±2.2 | 0.615 |
| | B 2.5+Sc 5 | 44.9±2.2 | 0.57 |
| | B 5+Sc 10 | 65.2±3.1 | 0.476 |
| | B 10+Sc 20 | 71.62±1.2 | 0.699 |
| | B 20+Sc 40 | 87±2 | 0.5 |
| | B 2.5+Sc 10 | 63.5±3.2 | 0.3842 |
| | B 5+Sc 20 | 69.35±2.2 | 0.573 |
| | B 10+Sc 40 | 78.7±3 | 0.683 |
| | B 20+Sc 80 | 87±3 | 0.725 |

Table 15

| Single dose (μm) | Combination dose (μm) | Inhibition of cell growth (%) | Combination Index (CI) |
|---|---|---|---|
| B 2.5 | | 5±2 | |
| B 5 | | 16.8±1.2 | |
| B 10 | | 38.4±2.1 | |
| B 20 | | 66.85±2 | |
| B 40 | | 83.1±2.1 | |
| Sc 2.5 | | 4.7±2.2 | |
| Sc 5 | | 12.97±3.1 | |
| Sc 10 | | 27.1±2.7 | |
| Sc 20 | | 66±2.9 | |
| Sc 40 | | 84.62±2.5 | |
| | B 5+Sc 5 | 22.1±2.1 | 1.46 |
| | B 10+Sc 10 | 51.67±2.3 | 1.33 |
| | B 20+Sc 20 | 85.7±2.1 | 0.956 |
| | B 40+Sc 40 | 97±2.5 | 0.7 |
| | B 2.5+Sc 5 | 19.2±3 | 1.2 |
| | B 5+Sc 10 | 47.7±2.3 | 1 |
| | B 10+Sc 20 | 75.62±2.2 | 0.99 |
| | B 20+Sc 40 | 92±3 | 0.97 |
| | B 2.5+Sc 10 | 48.6±3 | 0.87 |
| | B 5+Sc 20 | 77.33±2.2 | 0.826 |
| | B 10+Sc 40 | 89.7±3 | 0.9559 |
| | B 20+Sc 80 | 98.5±2 | 0.588 |

Table 16

| Single dose (μm) | Combination dose (μm) | Inhibition of cell growth (%) | Combination Index (CI) |
|---|---|---|---|
| B 2.5 | | 0.8±2 | |
| B 5 | | 13.02±2.5 | |
| B 10 | | 51.02±2.2 | |
| B 20 | | 64.35±2.2 | |
| B 40 | | 93±2.5 | |
| Sc 2.5 | | 5±2.5 | |
| Sc 5 | | 20.71±3.2 | |
| Sc 10 | | 71±2.2 | |
| Sc 20 | | 87.5±2.5 | |
| Sc 40 | | 95.57±2.2 | |
| | B 5+Sc 5 | 71.65±2.3 | 0.65 |
| | B 10+Sc 10 | 89.7±2.2 | 0.75 |
| | B 20+Sc 20 | 96.5±2.5 | 0.917 |

(continued)

| Single dose (μm) | Combination dose (μm) | Inhibition of cell growth (%) | Combination Index (CI) |
|---|---|---|---|
| | B 40+Sc 40 | 99±2 | 1 |
| | B 2.5+Sc 5 | 61.8±2 | 0.62 |
| | B 5+Sc 10 | 85.7±3.5 | 0.7 |
| | B 10+Sc 20 | 95±2.5 | 0.8 |
| | B 20+Sc 40 | 98.2±2.1 | 1 |
| | B 2.5+Sc 10 | 72.8±2.1 | 0.867 |
| | B 5+Sc 20 | 92.5±2.1 | 0.88 |
| | B 10+Sc40 | 97.2±1.7 | 1.1 |
| | B 20+Sc 80 | 99.5±2 | 1 |

Table 17

| Single dose (μm) | Combination dose (μm) | Inhibition of cell growth (%) | Combination Index (CI) |
|---|---|---|---|
| B 2.5 | | 7.2±5 | |
| B 5 | | 22.8±6 | |
| B 10 | | 50.9±5 | |
| B 20 | | 69.9±5 | |
| B 40 | | 92.9±7 | |
| Sc2.5 | | 2±5 | |
| Sc 5 | | 18±3 | |
| Sc 10 | | 39±7 | |
| Sc 20 | | 72±9 | |
| Sc 40 | | 91.8±5 | |
| | B 5+Sc 5 | 58±3.9 | 0.74 |
| | B 10+Sc 10 | 79±5 | 0.89 |
| | B 20+Sc 20 | 91±5 | 1 |
| | B 40+Sc 40 | 98±7 | 0.96 |
| | B 2.5+Sc 5 | 52±3 | 0.61 |
| | B 5+Sc 10 | 69±5 | 0.858 |
| | B 10+Sc 20 | 87±5 | 0.99 |
| | B 20+Sc 40 | 97±3 | 0.91 |
| | B 2.5+Sc 10 | 72±3 | 0.65 |
| | B 5+Sc 20 | 82.8±5 | 0.97 |
| | B 10+Sc 40 | 95±3 | 1 |
| | B 20+Sc 80 | 99±5 | 0.91 |

Table 18

| Single dose (μm) | Combination dose (μm) | Inhibition of cell growth (%) | Combination Index (CI) |
|---|---|---|---|
| B 2.5 | | 7.5±2.7 | |
| B 5 | | 22.7±2.2 | |
| B 10 | | 41.8±2.7 | |
| B 20 | | 60.6±3.2 | |
| B 40 | | 89.18±2.1 | |
| Sc 2.5 | | 2.25±2.7 | |
| Sc 5 | | 8.64±3.2 | |
| Sc 10 | | 37±2.2 | |
| Sc 20 | | 64.2±2.7 | |
| Sc 40 | | 88.5±2.5 | |
| | B 5+Sc 5 | 52.02±2 | 0.743 |
| | B 10+Sc 10 | 72.2±2.3 | 0.88 |
| | B 20+Sc 20 | 89.6±2 | 0.87 |
| | B 40+Sc 40 | 95.8±2.2 | 0.97 |
| | B 2.5+Sc 5 | 18±2.7 | 1.38 |
| | B 5+Sc 10 | 69.3±2.7 | 0.72 |
| | B 10+Sc 20 | 82.3±3.2 | 0.94 |
| | B 20+Sc 40 | 95.7±2.5 | 0.76 |
| | B 2.5+Sc 10 | 58±3 | 0.78 |
| | B 5+Sc 20 | 77±2.1 | 0.95 |
| | B 10+Sc 40 | 92.2±2.3 | 0.928 |
| | B 20+Sc 80 | 98.1±3 | 0.8 |
| B=baicalein; Sc=scutelarin | | | |

Example 5

Synergistic anti-tumor effects of a combination of baicalein and scutellarin on solid tumor transplant animal models

C57BL/6 female mice (weight approximately 20 g) were used in this study. Murine melanoma cell line B16 cells, $2\times10^6$ tumor cells were implanted s.c. into the armpit of each mice after two passenges in vitro cultured. On the next day after implantation, mice were randomly grouped at 10 per group and received 0.1ml/10g weight of the drugs. Anti-tumor effect studies were evaluated using a combination dosing of baicalein (Kunming Tongchi Pharmaceutical Co., Ltd.) and scutellarin (Kunming Longjin Pharmaceutical Co., Ltd). The number of mice of each group was 10. Mice were weighed before and post the treatment. Animals were killed on day 15, and the tumors were removed and weighted. The tumor growth inhibition was determined as; inhibition rate=(1-tumor weight of treatment group/ tumor weight of control group) ×100%, tumor growth inhibition rate was calculated, the data were analyzed by t-test. The results is shown in Table 19.

Table 19

| Group | Dose mg/kg | Route and Schedule | Weight Change % | Final Tumor WT.(g) Mean±SD, | Tumor Growth Inhibition % | Number of mice through 15days/total number |
|---|---|---|---|---|---|---|
| Control | - | | <0 | 2.18±0.15 | | 10/10 |

(continued)

| Group | Dose mg/kg | Route and Schedule | Weight Change % | Final Tumor WT.(g) Mean±SD, | Tumor Growth Inhibition % | Number of mice through 15days/total number |
|---|---|---|---|---|---|---|
| B | 30 | i.p.; day1-10 | <5 | 1.72±0.09 | 21.1 | 10/10 |
| Sc | 25 | i.p.; day1-10 | <5 | 1.81±0.11 | 17 | 10/10 |
| Sc | 50 | i.p.; day1-10 | <5 | 1.72±0.08 | 21.1 | 10/10 |
| Sc | 100 | i.p.; day1-10 | <5 | 1.48±0.12 | 32.1 | 10/10 |
| Sc | 200 | i.p.; day1-10 | <5 | 1.22±0.09 | 44 | 10/10 |
| B+ | 30+ | i.p.; day1-10 | <5 | 1.62±0.08 | 24.3* # | 10/10 |
| Sc | 25 | i.p.; day1-10 | | | | |
| B+ | 30+ | i.p.; day1-10 | <5 | 1.51±0.12 | 30.7** # | 10/10 |
| Sc | 50 | i.p.; day1-10 | | | | |
| B+ | 30+ | i.p.; day1-10 | <5 | 1.2±0.09 | 45** # | 10/10 |
| Sc | 100 | i.p.; day1-10 | | | | |
| B+ | 30+ | i.p.; day1-10 | <5 | 1.07±0.08 | 51**# | 10/10 |
| Sc | 200 | i.p.; day1-10 | | | | |
| 5-FU | 10 | s.c.;day1-10 | <5 | 1.35±0.1 | 38 | 10/10 |

B=baicalein, Sc=scutellarin
*P<0.05 comparison between the baicalein-treated and a combination-treated group,
** P<0.01 comparison between the baicalein-treated and a combination-treated group.
# P<0.01 comparison between the scutellarin -treated and a combination-treated group.

The study showed that significantly enhancement of anti-tumor activity by using baicalein and scutellarin combination. Treatment group of a combination of baicalein and scutellarin at various doses, such as baicalein+scutellarin, 30+50 mg/kg; baicalein+scutellarin, 30+100mg/kg; baicalein+scutellarin, 30+200 mg/kg demonstrated a significant statistical difference compared to when using ether baicalein or scutellarin alone. In addition, the group of baicalein+scutellarin at a dose of 30+25 mg/kg, also showed some enhancement of anti-tumor activity, but not clearly better than other three dose groups. Date of animal weight and weight change indicated there was no enhancement of toxicity in each combination dosing group.

The ratio of baicalein to scutellarin effect between 1:0.5 and 1:4 (molar ratio) started to show the synergistic anti-tumor effects, and reach the plateau between 1:1 and 1:4. The animal modle data were consistent with those of in vitro studies. Therefore, a combination baicalein and scutellarin has synergistic anti-tumor effects on solid tumor.

Example 6

Synergistic anti-tumor effects of baicalein and scutellarin combination on transplant blood tumor models

L1210 cells on the growth phase were injected at $1\times10^5$ cells via I.P. into BDF1 female mice (weight approximately 20 g). The next day of implantation, mice were grouped, and received an injection of 0.1ml/10g weight of the drug. Anti-tumor effects were evaluated using single or combination dosing of baicalein (Kunming Tongchi Pharmaceutical Co., Ltd.) and scutellarin (Kunming Longjin Pharmaceutical Co., Ltd). The number of mice of each group was 10. Mice were weighed before and after the treatment. Survival time of each mouse was recorded. The results of changes in increasing of life span of mice were used in assessment of response. The increase of life span was determined as; increase of life span rate=(median survival time of treated animals/ median survival time of control animals-1)×100%, the data were analyzed by t-test. The results is shown in Table 20.

Table 20

| Group | Dose mg/kg | Route and Schedule | ILS(%) | Number of mice Through surviving 15days/total number |
|---|---|---|---|---|
| Control | - | | | 10/10 |
| B | 30 | i.p.; day1-10 | 31.6 | 10/10 |
| Sc | 25 | i.p.; day1-10 | 11.9 | 10/10 |
| Sc | 50 | i.p.; day1-10 | 26 | 10/10 |
| Sc | 100 | i.p.; day1-10 | 37 | 10/10 |
| Sc | 200 | i.p.; day1-10 | 51 | 10/10 |

(continued)

| Group | Dose mg/kg | Route and Schedule | ILS(%) | Number of mice Through surviving 15days/total number |
|---|---|---|---|---|
| B+ | 30+ | i.p.; day1-10 | 36* # | 10/10 |
| Sc | 25 | i.p.; day1-10 | | |
| B+ | 30+ | i.p.; day1-10 | 50**# | 10/10 |
| Sc | 50 | i.p.; day1-10 | | |
| B+ | 30+ | i.p.; day1-10 | 53.7**# | 10/10 |
| Sc | 100 | i.p.; day1-10 | | |
| B+ | 30+ | i.p.; day1-10 | 64.8**# | 10/10 |
| Sc | 200 | i.p.; day1-10 | | |
| 5-FU | 10 | s.c..; day1-10 | 79 | 10/10 |

B=baicalein,
Sc=scutellarin
ILS=increase of life span
*P<0.05 comparison between the baicalein-treated and a combination-treated group,
** P<0.01 comparison between the baicalein-treated and a combination-treated group.
# P<0.01 comparison between the scutellarin -treated and a combination-treated group.

The study showed that significantly enhancement of anti-tumor activity by using baicalein and scutellarin combination. Treatment group of a combination of baicalein and scutellarin at various doses, such as baicalein+scutellarin, 30+50 mg/kg; baicalein+scutellarin, 30+100 mg/kg; baicalein+scutellarin, 30+200 mg/kg demonstrated a significant statistical difference compared to when using ether baicalein or scutellarin. In addition, the group of baicalein+scutellarin at a dose of 30+25 mg/kg, also showed some enhancement of anti-tumor activity, but not clearly better than other three dose groups. Data of animal weight change indicated there was no enhancement of toxicity in each combination dosing group.

The ratio of baicalein to scutellarin effect between 1:0.5 and 1:4 (molar ratio) started to show the synergistic anti-tumor effects, and reach the plateau between 1:1 and 1:4. The animal model data were consistent with those of in vitro studies. Therefore, a combination baicalein and scutellarin has synergistic anti-tumor effects on blood tumor.

All documents cited for the disclosure of the invention are hereby incorporated hereto in their entirety by reference. It should be understood, however, it would be obvious to those skilled in the art that various other changes and modifications can be made without departing from the spirit and scope of the invention. It is therefore intended to cover in the appended claims all such changes and modifications that are within the scope of this invention.

## Claims

**1.** A pharmaceutical composition having synergistic anti-tumor activities, comprising baicalein to a compound of formula I, **characterized in that** the molar ratio of baicalein and the compound of formula I is between 1:1 and 1:4,

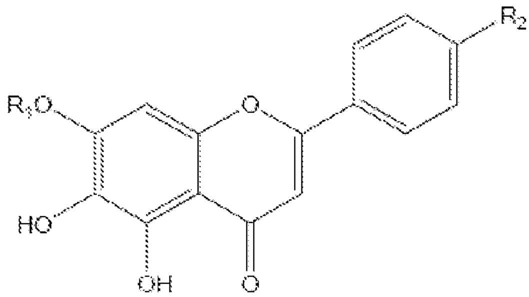

(I)

wherein
R1 is a glucuronic acid group, and
R2 is a hydrogen or a hydroxyl group.

2. The pharmaceutical composition of claim 1, wherein the tumor is a solid tumor or blood tumor.

3. The pharmaceutical composition of claim 1, wherein the tumor is selected from melanoma, liver cancer, colon cancer, lung cancer, gastric cancer, esophageal cancer, breast cancer, prostate cancer or leukemia.

4. A method of producing the pharmaceutical composition according to any one of claims 1-3, comprising the step of mixing baicalein with at least one compound

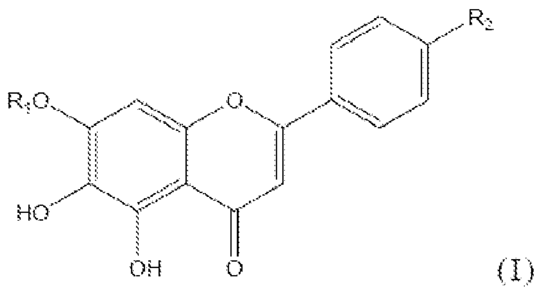

(I)

wherein
R1 is a glucuronic acid group, and
R2 is a hydrogen or hydroxyl group.

5. The method of claim 4, further comprising the step of formulating the mixture into a suitable dosage form by adding pharmaceutically acceptable additives.

6. Use of the pharmaceutical composition of any one of claim 1-3 in the preparation of a medicament for tumor treatment.

7. The use of claim 6, wherein the tumor is a solid tumor or a blood tumor.

8. The use of claim 6, wherein the tumor is selected from melanoma, liver cancer, colon cancer, lung cancer, gastric cancer, esophageal cancer, breast cancer, prostate cancer or leukemia.

| INTERNATIONAL SEARCH REPORT | International application No. PCT/CN2006/000088 |
|---|---|

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)

IPC⁸: A61K, A61P

Documentation searched other than minimum documentation to the extent that such documents are included in the fields searched

CPRS

Electronic data base consulted during the international search (name of data base and, where practicable, search terms used)

WPI,EPODOC,PAJ, CNKI(CN), Chinese Pharmaceutical Abstract, CA, MEDLINE,EMBASE, baicalin,baicalein, scutellarin,cancer,synergistic,

C. DOCUMENTS CONSIDERED TO BE RELEVANT

| Category* | Citation of document, with indication, where appropriate, of the relevant passages | Relevant to claim No. |
|---|---|---|
| X | CN 1442134 A(HANGZHOU HUADONG MEDICINE GROUP CORP) (17.Sep.2003)(17.09.2003), page 7, example 4 | 1-8 |
| X | CN 1442135 A (HANGZHOU HUADONG MEDICINE GROUP CORP)(17.Sep.2003)(17.09.2003), page 7, example 4 | 1-8 |
| A | CN 1381236 A (SHENGWUGU SCI & TECHNOLOGY CO LTD SHENZH) (27.Nov.2002) (27.11.2002) see the whole document | 1-8 |
| | | |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 23.Mar.2006(23.03.2006) | 06 APR 2006 (06 · 04 · 2006) |
| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br>HUANG Yijie<br>Telephone No. 86-10-62085252 |

Form PCT/ISA /210 (second sheet) (April 2005)

**INTERNATIONAL SEARCH REPORT**
Information on patent family members

International application No.
PCT/CN2006/000088

| Patent Documents referred in the Report | Publication Date | Patent Family | Publication Date |
|---|---|---|---|
| CN 1442134 A | 17.09.2003 | none | |
| CN 1442135 A | 17.09.2003 | none | |
| CN 1381236 A | 27.11.2002 | none | |

Form PCT/ISA /210 (patent family annex) (April 2005)

## INTERNATIONAL SEARCH REPORT

International application No.
PCT/CN2006/000088

CLASSIFICATION OF SUBJECT MATTER

A61K 31/352 (2006.01) i
A61K 31/7048 (2006.01) i
A61P 35/02 (2006.01) i
A61P 35/04 (2006.01) i

Form PCT/ISA /210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9323033 A


### Non-patent literature cited in the description


- Chemotherapy: Synergism and Antagonism. **JOSEPH R. BERTINO ; TING-CHAO CHOU.** Encyclopedia of Cancer. Academic Press, Inc, 1996
- **R. LAROCK.** Comprehensive Organic Transformations. VCH Publishers, 1989
- **T.W. GREEN ; P.G.M. WUTS.** Protective Groups in Organic Synthesis. John Wiley and Sons, 1999
- **L. FIESER ; M. FIESER.** Fieser and Fieser's Reagents for Organic Synthesis. John Wiley and Sons, 1994
- Encyclopedia of Reagents for Organic Synthesis. John Wiley and Sons, 1995
- **NEGRIN et al.** *Biomaterials,* 2001, vol. 22 (6), 563